# EUROPEAN PATENT APPLICATION

(11) **EP 2 789 331 A1**
(43) Date of publication of application: **15.10.2014**
(21) Application number: 14175933.2
(22) Date of filing: 12.02.2004
(51) Int. Cl.: A61K 9/127, A61K 31/79

(54) **Use of PVP-Iodine liposomes for treatment of herpes**

(30) Priority: 24.02.2003 EP 03003282
(62) Divisional of application: 04710376.7
(71) Applicant: Euro-Celtique S.A., 1653 Luxembourg (LU)
(72) Inventor: REIMER, Karen, 65582 Hambach (DE); FLEISCHER, Wolfgang, 55218 Ingelheim (DE)
(74) Representative: Braun, Nils

(57) **Abstract**

The invention concerns a method for the production of a pharmaceutical preparation for the treatment of Herpes forms that is characterized in, that the preparation comprises at least one antiseptic compound associated with a particular carrier.

## Description

The present invention concerns the use of PVP-iodine liposomes for the treatment of herpes. The present invention also concerns a method for producing a pharmaceutical preparation for the treatment of herpes virus-induced skin lesions, the preparation comprising at least one antiseptic compound in a pharmaceutically effective amount combined with a particulate, pharmaceutically acceptable carrier.

The term herpes usually refers to a viral inflammatory condition of the skin and mucosa which is accompanied by the formation of small and painful blisters. The viral infectious conditions which commonly go under the general name of herpes are essentially caused by two different virus types of the herpes virus group, namely the herpes simplex virus and the herpes zoster virus.

Herpes simplex viruses (HSV) are enveloped, cubic DNA viruses that usually infect the termini of nerve cells via the mucosas of the mouth (predominantly HSV Type 1) or the genitalia (predominantly HSV Type 2). They are transported within the nerve cells by retrograde axonal transport to the nerve cell bodies in the ganglia. After one to two days active productive infection begins which peaks on the fourth day and is restricted to a minimum from the sixth day onwards (probably through cellular defence mechanisms). While the first symptoms of skin irritation and blistering appear from the sixth day after infection onwards, virus secretion continues up to the tenth day.

The virus remains within the nerve cells even after the skin and mucosa infections have healed. Frequently, a reactivation of the virus and correspondingly new skin irritations occur due to stress-creating factors, such as e.g. sun exposure, fever, hormonal influences, a general weakening of the immune defence, stomach upsets and gastro-intestinal disorders, menstruation and traumata. These recurring symptoms, which are due to herpes simplex virus Type 1 and Type 2 infections, are named after the corresponding triggering event also as *Herpes febrilis, Herbes solaris, Herpes manstrualis, Herpes traumatica* etc. HSV viruses Type 1 and Type 2 may also be responsible for the occurrence of *Herpes corneae* (also known as Keratoconjunctivitis herpetica). Severe forms of *Herpes corneae* are characterized by attack of the endothelium accompanied by with a disc-like turbidity of the cornea.

Depending on the part of the body affected, the different herpes virus-induced conditions may also be designated as *Herpes labialis, Herpes genitalis* or *Herpes corneae.*

Usually, nucleoside-analoga, such as e.g. ganciclovir, are used in the treatment of herpes forms tiggered by HSV Type 1 or HSV Type 2. These nucleoside-analoga are metabolised by HSV-thymidine kinase to toxic products which ultimately leads to the killing of infected cells. However, the use of nucleoside-analoga has the main drawback that these compounds may also be incorporated into the DNA of replicating cells and may act mutagenic in that way. Moreover, the use of nucleoside-analoga only aims at removing the causes of the symptoms of the herpes conditions, i.e. to repress the outbreak and spreading of the viral infection. An efficient treatment of the symptoms, i.e. healing of the painful blisters, is not possible with these compounds. Improvement of the symptoms using the nucleoside-analoga is rather a long term consequence.

Herpes zoster, generally known also as shingles, is caused by the varizella zoster virus (VZV). Primary infection with VZV leads to an exanthem with itchy blisters all over the body that subsequently slough and scar (chickenpox). This virus also persists in the ganglia cells of predisposed persons. Years or decades after healing of the varizella, the zoster can re-occur as a local reoccurrence of the condition in the form of extremely painful blisters in the supply region of certain nerves, particularly in the chest region. Here too, nucleoside-analoga are used in the treatment.

Due to the above-mentioned drawbacks of nucleoside-analoga, which are particularly relevant as far as the treatment of *Herpes genitalis* is concerned, there is a strong need for additional anti-viral agents that allow for an efficient treatment of herpes virus infections.

One compound for the treatment of herpes conditions which, because of its outstanding anti-viral properties deserves particular attention, is povidone-iodine. This iodine-releasing antiseptic is also known as polyvidone-iodone or PVP-iodone, i.e. poly(1-vinyl-2-pyrrolidin-2-on)-iodine complex. PVP-iodine has among other things the basic advantage that its application does not lead to the development of bacterial or viral resistances.

Various attempts concerning the use of PVP-iodine solutions for the treatment of herpes infections are known from prior art. As early as 1975 Friedrich et al. (Obstetrics and Gynecology, 45, 337-339) studied the effect of an alcoholic PVP-iodine solution commercially available under the name Betadine (in the US) or Betaisadonna (in Europe), for the treatment of symptoms as they appear during a Herpes genitalis infection. It was shown that the Betadine solution led to an improvement of lesions and a reduction of itchiness. This prior art document states that by applying the Betadine solution a healing of the symptoms of *Herpes genitalis* was achieved within one week. However, an assessment of the effectiveness of the Betadine solution is difficult, since in this study no control group was investigated.

The study carried out by Bullough et al. carried out in 1979 (Curr. Med. Res. Opin., 6, 175-177) also addressed the effectiveness of a 10% alcoholic PVP-iodine solution for the treatment of patients suffering from *Herpes genitalis.* The genital lesions healed within six to eight days. However, in some cases an improvement could only be observed after two weeks. Here too, an assessment of the effectiveness of the Betadine antiseptic paint solution is difficult because there was no control group.

The study carried out by Woodbridge et al. in 1977 (J. Int. Med. Res., 5, 378-382) also addressed the effect of the antiseptic Betadine solution containing 10% PVP-iodine. Patients suffering from Herpes simplex virus Type 1 or Herpes zoster infections were treated. An improvement of the symptoms of the Herpes simplex patients could be observed within one week. 13% of patients rated the observed improvement as average to bad, as against 18% of the patients who rated the improvement as excellent. Only 25% of patients with Herpes zoster rated the results as excellent.

The study of Simmons et al. from the year 1997 (Dermatology, 195 (suppl. 2), 85-88) also studied the efficacy of alcoholic Betadine PVP-iodine solution in the treatment of Herpes labialis. This study stands out because, in contrast to the prior art documents cited above, reliable statements with respect to the efficacy of the Betadine solution can be made since control groups were used. The efficacy of the Betadine solution was judged based on a comparison of smear tests taken before and after Betadine treatment regarding their ability to induce virus infections in cultures.

It was shown that Herpes labialis could be better treated with a Betadine solution compared to the other compounds investigated, to the extent that less viral pathogens could be detected in Herpes labialis blisters after treatment with the Betadine solution. With the Betadine solution, the detectable Herpes simplex virus discharge could be stopped in 60 to 64% of cases. However, this prior art provides no information regarding the efficiency of the treatment of symptoms, i.e. whether the Betadine solution accelerates healing of the blisters and relieves itchiness. This prior art also makes no statements as to the effectiveness of the Betadine solution with respect to skin irritations as caused by the Herpes zoster virus. Statements are only made regarding the altered virus load, i.e. regarding the causes of herpes conditions, but not regarding improvement of the patient's painful symptoms following a Betadine treatment.

The afore-mentioned soap solutions which are usually of a brown colour have the additional drawback that the treated skin may show coloured patches. Even though the colour may be removed by thorough washing, this is not acceptable where sensitive and already pre-damaged skin areas are to be treated.

Besides the afore-mentioned prior art documents there are a number of studies which also demonstrate the effect of PVP-iodine solutions, either in the form of aqueous or alcoholic solutions against Herpes simplex viruses. However, proof of antiviral effectiveness always relates to the ability of the PVP-iodine solutions to inactivate the viral pathogens in *in vitro* test systems. The effectiveness of these PVP-iodine solutions for the treatment of symptoms as skin lesions and itchiness, which subjectively are often more important for the patient, is not addressed by these prior art documents.

Kawana et al. ((1997), Dermatology, 195 (suppl. 2), 29-35) describe a comparison of different antiseptic compounds during inactivation of human viruses. The teaching of this document is that PVP-iodine is an effective antiviral compound when mixed as an aqueous solution with viral suspensions. After this treatment the viral suspensions are only capable to a limited extent of infecting cellular systems. The influence of PVP-iodine preparations for the treatment of skin lesions caused by Herpes simplex viruses is not discussed in this document. The influence of PVP-iodine on the healing of blisters caused by Herpes zoster viruses is also not addressed.

An article by Benevento et al. from 1990 (American Journal of Ophthalmology, 109, 329-333) describes an investigation of the effectiveness of variously concentrated PVP-iodine solutions with respect to Herpes simplex Type 2 viruses. This prior art also only deals with the antiviral *in vitro* activity of PVP-iodine. What was measured was antiviral activity of PVP-iodine solutions with regard to its ability to reduce the infectivity of Herpes simplex virus supernatants. The influence of PVP-iodine solutions on the healing of skin conditions caused by Herpes simplex and Herpes zoster viruses is not dealt with in this prior art document.

Liposomes are known pharmaceutical compound carriers and the administration of medicaments in liposomal form has been the subject of studies for quite some time. Even though PVP-iodine liposome preparations are known from e.g. European patent EP 0 639 373, these preparations have not been studied with respect to their effectiveness in the treatment of skin irritations caused by Herpes simplex or Herpes zoster viral infections.

An article by Wutzler et al. ((2000), Ophthalmic Res., 32, 118-125) describes the antiviral activities of eye drops comprising PVP-iodine liposomes. The antiviral activity is measured solely by *in vitro* measurements, i.e. the ability of the PVP-iodine liposome complexes to reduce the infectivity of Herpes simplex virus supernatants. The use of PVP-iodine liposome complexes in the treatment of skin irritations and lesions, as they occur with Herpes simplex and Herpes zoster conditions is not the subject of this publication. The teaching of the afore-mentioned article is rather concerned with the fact that the PVP-iodine liposome complexes may be used as prophylaxis against endophthalimitis and Herpes corneae in the course of ophthalmological surgery.

Another article by Wutzler et al. dating from 2002 (Antiviral Research, 54, 89-97) is again only concerned with the antiviral activity and the cytotoxicity of PVP-iodine liposome complexes. Here too, the antiviral activities of PVP-iodine liposome complexes are measured only by the afore-mentioned *in vitro* tests. The putative effectiveness of such PVP-iodine liposome complexes in the treatment of e.g. *Herpes labialis* is not dealt with in this publication at all. Rather, this publication teaches that the afore-mentioned PVP-iodine liposome complexes can be primarily used for treatment in connection with bacterial and viral kerato-conjunctivitis and for the treatment of viral infections of the respiratory tract.

None of the afore-mentioned prior art documents contains hints for the production of liposomes containing PVP-iodine for the treatment of skin lesions and itchiness caused by herpes infections.

The use of antiseptic and/or agents promoting wound-healing for external application on humans and animals is already disclosed in EP 0 639 373. In particular, liposome preparations of PVP-iodine are shown therein to be topically application to the external parts of the eye mainly for purposes of complete disinfection. These preparations generally take the form of a cream, an ointment, a lotion, a gel or a drop formulation.

Liposomes are well-known drug or compound carriers and thus the application of medicaments in liposomal form has been the subject of investigation for quite some time. An overview concerning the administration of compounds in liposomal form to the skin is provided by the review "Targeted delivery to the pilosebaceous unit via liposomes (Lauer, A.C. et al. (1996), Advanced Drug Delivery Reviews, 18, 311-324). This review describes the physical-chemical characterisation of liposomal preparations and their therapeutic applications for the treatment of the pilosebaceous unit. Compounds that have been investigated for delivery by liposomes include e.g. anti-cancer agents, peptides, enzymes, anti-asthmatic and anti-allergic compounds and, as mentioned above, also antibiotics.

Despite the fact that the prior art contains many hints for the use of liposomal preparations for the treatment of viral infections of the eye and respiratory tract, there seems to be no prior art concerning liposomes or other particles as carriers for antiseptic compounds for the treatment of skin lesions of itchiness due to herpes infections. In view of the fact that liposomes have been known in the art for a long time as drug carriers and that PVP-iodine has also been known for quite some time (some of the afore-mentioned prior art dates back to the seventies) as a compound for the treatment of herpes infections, there seems to be a strong reluctance in the prior art to use antiseptic compounds in the form of liposomes for the treatment of skin symptoms attributable to herpes infections. In general, preparations that do not comprise liposomes or other compounds such as nucleoside-analoga seem to be preferred, in spite of the disadvantages set out above.

In the treatment of skin lesions as it occurs due to Herpes simplex and Herpes zoster viruses, the accessibility of the surface of the damaged skin parts is decisive for the effectiveness of the compounds used in treatment. One prior art document, namely the publication by Taylor et al. from 1987 (Journal of Hospital Infection, 9, 22-29) deals with the efficacy of PVP-iodine solutions in test systems where especially the antiviral activity of antiseptic compounds on surfaces is tested. Interestingly, this prior art document shows that PVP-iodine solutions are significantly less efficient in killing herpes viruses that have been immobilized and dried on surfaces in comparison to the other tested antiseptic solutions such as hypochloride-solutions or alkaline glutaraldehyde.

It is clear from the above statements that the prior art does not disclose the use of PVP-iodine formulations which, in treating Herpes simplex or Herpes zoster infections, lead to a reduction of the virus load and allow at the same time for an efficient treatment of symptoms such as painful skin lesions, blisters and itching caused by the infections.

One objective of the present invention is to provide for a well-tolerated, easily applicable pharmaceutical preparation for the topical treatment of the causes and symptoms of herpes infections, which renders possible a lasting, efficient and scar-free treatment of the symptoms such as skin lesions, painful blistering and itchiness. This and further objectives of the present invention, which will become clear from the description, are solved by the subject-matter of the independent claim. Preferred embodiments of the present invention are defined by the dependent claims.

It has surprisingly been found that the preparations according to the present invention comprising an antiseptic compound, such as PVP-iodine, combined with particulate carriers, such as liposomes, can be ideally used to efficiently treat symptoms as skin lesions occurring as a result of Herpes simplex and Herpes zoster infections. Treatment of such skin lesions may include the efficient and fast healing of painful blisters. According to the invention, this use of the afore-mentioned preparations has the advantage that in comparison to preparations known from the prior art, a faster, more efficient and scar-free healing of the painful blisters takes place. The preparations according to the invention are also ideally suited for the treatment of symptoms occurring in the course of infections with the various forms of herpes viruses mentioned above, because the liposomal formulation has a positive effect on membrane stability of the skin parts to be treated. Moreover, the liposomal formulation most probably induces an efficient and lasting penetration of the PVP-iodine complexes, also into deeper layers of the afflicted skin.

The composition of the liposomes, the concentration of the active compound(s) and methods for producing the PVP-iodine liposomes or preparations comprising an antiseptic compound in a pharmaceutically effective amount combined with the particulate carriers are presented below. If, for purposes of demonstration, liposomes and PVP-iodine are mentioned, the person skilled in the art is well aware that other carriers and other antiseptics may be formulated in analogous manner and may thus also be used for the same purposes.

One object of the present invention is a method for producing a pharmaceutical preparation for the use of at least one antiseptic compound for the treatment of the causes and symptoms of herpes infections, the preparation comprising the compound(s) in a pharmaceutically effective amount combined with a particulate, pharmaceutically acceptable carrier.

Surprisingly it was found that the pharmaceutical preparations according to the invention comprising an antiseptic compound, such as PVP-iodine, associated with particulate carriers, such as liposomes, can be ideally used for the topical treatment of the causes, and especially the symptoms of Herpes simplex and Herpes zoster infections, such as skin lesions, painful blisters and intense itchiness. According to the invention, the novel use of particle-containing preparations comprising antiseptic compounds for the treatment of the symptoms occurring during herpes infections has moreover the surprising advantage that faster healing of the skin lesions caused by herpes viruses is rendered possible, in comparison to preparations known in the art.

The invention is thus based on the surprising fact that particulate carriers, especially liposomes, are exceptionally well-suited as carriers for antiseptic agents, especially for PVP-iodine, for the application and treatment of herpes infections.

The invention is thus based on the surprising finding that particulate carriers, especially liposomes, are exceptionally well-suited as carriers for antiseptic agents, especially for PVP-iodine for the application and treatment of mild and severe forms of atopic dermatitis and other forms of dermatitis mentioned above.

The preparations according to this invention permit protracted release of the compound(s) and render possible a lasting and local activity at the desired spot by interacting with the respective skin cell surfaces. Without wanting to by bound to a specific scientific theory, it is assumed that the outstanding effect of the PVP-iodine liposomes according to the invention is due to the deeper penetration of the liposomes into the damaged skin areas compared to conventional preparations. In this way, the compound(s) is/are transported more efficiently to the damaged skin parts. However, it is then surprising that such a radically effective substance class such as antiseptics does not impinge on the healing process of the especially sensitive and damaged tissue and can even suppress the formation of scar tissue, neoplasms, intergrowth etc.. This may be due to the granulation and epithelium-forming effect of the liposomal preparations.

In the context of the present invention the term "herpes" comprises those inflammatory skin conditions that can be caused by the various known herpes viruses. The term "herpes" comprises in particular the various herpes virus induced conditions such as *Herpes labialis, Herpes genitalis, Herpes febrilis, Herpes solaris*, *Herpes menstrualis* and/or *Herpes traumatica.* Likewise in the context of the present invention those conditions are called herpes which are caused by the Varizella zoster virus. This comprises e.g. shingles, facial erysipelas or chickenpox. It is currently not intended to use preparations according to the invention for treatment of symptoms of herpes infections on the eye. Preparations according to the invention are used mainly for treatment of skin lesions on the face, lips, chest and back area and on the extremities as they occur as a result of herpes infections.

In the context of the present invention, antiseptic compounds first and foremost comprise compounds designated as antiseptics and used as such in the prior art. According to the invention these compounds especially comprise such disinfecting compounds that are pharmaceutically acceptable and can be used for the treatment of the various forms of atopic dermatitis mentioned above by topical application, as long as they have a formulation according to the invention. The antiseptic compounds preferably comprise, among other things, oxygen-releasing or elementary halogene-releasing compounds as well as metal compounds, such as silver and mercury compounds. More preferably, the antiseptic compounds according to the invention comprise halogene-releasing compounds such as iodine, iodine complexes and PVP-iodine which contain the iodine in elementary form.

In the context of the present invention the term "pharmaceutically effective amount" refers to an amount of the antiseptic(s) in the preparations that is sufficient for treating the various afore-mentioned herpes forms efficiently.

According to the invention, the preparations according to the invention can be used to treat the various forms of herpes, so that upon topical application symptoms as the painful blisters developing in the course of herpes infections and other skin lesions regress and heal substantially completely, i.e. scar-free, compared to the application of pharmaceutical preparations known from the prior art.

This effect is due to the surprising and unexpected fact that through use of preparations according to the invention, such as PVP-iodine-containing liposomes, a hyperkeratosis and uncontrolled growth of tissue can be avoided. The serious functional and cosmetically relevant skin damage that are one threatening consequence of the uncontrolled growth during the formation of new skin tissue can thus be prevented. Cosmetically, the scar-free and fast healing is particularly important, since the blisters and varizella typically affecting the face are experienced by the affected persons as extremely unpleasant. The fact that the use of e.g. PVP-iodine-containing liposomes allows such an efficient treatment of the various forms of herpes was especially surprising, since it could not be expected that by using preparations comprising only one active compound, the various complex reasons underlying the different herpes infections could be simultaneously and efficiently treated by topical applications without scar tissue occurring or remaining and without causing side effects.

PVP-iodine liposomes according to the present invention may therefore also be used for cosmetic purposes.

Moreover, PVP-iodine liposomes according to the invention ensure that the damaged skin parts remain germ-free and comprise additionally (and in addition retain) sufficient moisture to ensure sufficient healing of the damaged skin.

Using preparations according to the invention, the formation of scar tissue on the skin can be reduced and hyperkeratosis can be completely repressed. Intergrowth, or the formation of neoplasms that lead to scarring are significantly reduced upon use of e.g. PVP-iodine-containing liposomes for the treatment of various forms of herpes. Moreover, in the course of herpes treatment, the use of the afore-mentioned preparations also effects an efficient killing of the bacterial infections that are frequently a major side effect of herpes infections. Thus, antibiotics do not have to be administered as a supplementary therapy so that the danger of resistance development does not exist.

Due to the excellent antiviral effectiveness of the pharmaceutical preparations according to the invention, the spread of the virus and subsequent infections are prevented during the herpes treatment Thus, preparations according to the invention are clearly superior to preparations comprising only cosmetic substances and/or agents that promote wound-healing during the treatment of herpes infections.

The preparations according to the invention may also be used for treating the symptoms of the various forms of herpes such that a cosmetically acceptable and satisfying result is achieved for the affected persons. This aspect of the invention may also be designated as cosmetic remodelling.

Preparations according to the invention whose use permits the efficient treatment of the various forms of herpes can be produced by loading liposomes with PVP-iodine according to methods known in the art. The nature or composition of the liposomes is generally not decisive for the treatment success and can vary. The liposomal preparation, as for example described in EP 0 639 373, can be administered in different forms including, e.g. an ointment, a cream, a spray, a lotion, a solution, a suspension, a dispersion or a gel. The disclosure of EP 0 639 373 is incorporated herein by reference.

Preferably, the liposome-forming material is selected so that it does not react substantially or hardly reacts at all with the antiseptic compounds. One will therefore try to keep the content of chemically reactive substances as low as possible, if the antiseptic might be a reaction partner. If the antiseptic can release oxygen or halogen atoms, as is the case with PVP-iodine, one will use e.g. cholesterol which has a reactive double bond only in small amounts, if at all. In any case, one will choose the amount of such potentially reactive liposome-forming substances such that the pharmaceutical preparations have a sufficient storage stability of at least one, or even of at least two years (in compliance with statutory regulations). The storage conditions may comprise a temperature range of approximately -20 °C to approximately 60 °C.

The preparations according to this invention often contain the active compound(s), such as PVP-iodine, encapsulated in the particulate carrier, especially in liposomes. However, it may also occur that there is an amount of compound not contained inside the carrier. The compound(s) may also be associated with the surface of the particle carriers as e.g. liposomes.

In one embodiment of the invention the major part or even the whole amount of the active compund(s) may be located outside the particulate carriers as e.g. liposomes.

The preparations according to the invention then may show a marked initial effect which is observed in addition to the slower, protracted release of the active agent from the carrier. This effect is especially observed where the carrier comprises liposomes. Without wishing to be bound to any theoretical explanation, it is presently assumed that in addition to the active agent encapsulated inside the liposomes, some active agent is present outside of the liposomes, and probably loosely bound to the outer surfaces of the liposomes. This could be due to complex association of active compound molecules with the liposomal membrane, or it could be due to active compound molecules forming a layer on the liposomal surface, which layer partly or even fully coats the liposome externally. The type and amount of this initial compound effect can e. g. be influenced by choice of the concentration parameters.

In the context of the present invention, protracted or prolonged release means that the active compound(s) is/are released form the pharmaceutical preparation over a time period between 1 to 24 hours.

The association of antiseptic compounds with liposomes, i.e. that active compounds can be included in the interior of liposomes or, depending on the circumstances, can also associate with the surfaces, depends among other things on the components used for formation of the liposomes.

In a preferred embodiment, preparations according to the invention used for the treatment of the various forms of herpes can comprise, besides the antiseptic compound also other anti-inflammatory agents and agents promoting wound-healing.

These additional anti-inflammatory comprise e.g. phenolic compounds, detergents, alcohols, organic disinfectants including among other things formaldehye-releasing compounds, phenolic compounds including alkyl and aryl phenolic compounds, as well as halogenated phenolic compounds, chinolines, acridines, hexahydropyrimidines, quaternary ammonia compounds, iminium salts and guadinines. Agents promoting wound-healing comprise those substances that have been described in the literature for such applications. Such compounds comprise substances that are known for promoting the formation of epithelial tissue. These include vitamins, particularly from the vitamin B group, alantoin, some azulenes, etc.

In some embodiments of the present invention, preparations according to the present invention comprise antiseptic compounds, preferably PVP-iodine, and may also comprise compounds such as agents promoting wound-healing or anti-inflammatory compounds.

Inventive preparations can also contain other customary agents, including adjuvants and additives, antioxidants, conserving agents or consistency-forming agents such as viscosity-adjusting additives, emulgators, etc. The person skilled in the art will select these adjuvants and additives in such a way that the ability of preparations, substantially consisting of particulate carriers such as liposomes and antiseptic compounds such as PVP-iodine, to efficiently treat the various forms of herpes, is not impaired. Additives may also comprise salts that allow for the regeneration of the active compound, such as the released halogen atom in case of halogen-releasing compounds. In the case of PVP-iodine such an additive may be KIO₃. Other additives that mediate or enhance penetration of the liposomes into the skin may also be part of the inventive preparations. Such additives comprise e.g. DMSO.

The amphiphilic substances generally known in prior art to form liposome membranes can be employed in the context of the invention as long as they are pharmaceutically acceptable for the intended application. Presently, liposome-forming systems comprising lecithin are preferred. Such systems can comprise hydrogenated soy bean lecithin besides cholesterol (if suitable despite its reactivity) and disodium succinatehexahydrate. Preferably one will make sure that the liposome-forming materials do not show any reactivity with the antiseptics in order to ensure the required storage stability of commercial products. Due to its double-bond reactivity high cholesterol contents will be avoided where it is to be formulated in connection with oxygen-releasing or halogen-releasing antiseptics (such as PVP-iodine). It is presently specifically preferred to use hydrogenated soy bean lecithin as the sole membrane-forming agent. Commercially available products such as Phospholipon® 90 H (Aventis, Germany) or Lipoid S100-3 (Lipoid GmbH, Germany) are also preferred.

As can be taken from the review of Lauer A.C. et al. 1995 (*vide supra*) phospholipid-based liposomes may also be generally used for production of liposomes that discharge their cargo into the skin. According to this review, the use of non-ionic liposomes, which can be formed with phosphatidylcholin, is also an option. The presence of sebum in the hair follicle may be relevant for the choice of components that the liposomes are formed from. Other components that may be used for the formation of micelles are also known to the person skilled in the art and may be used for the production of preparations according to the invention.

The known prior art methods for forming liposome structures can generally be used in the context of the invention. Broadly, these methods comprise mechanical agitation of a suitable mixture containing the membrane-forming substance and water or an aqueous solution. Filtration through suitable membranes is preferred in order to form a substantially uniform liposome size.

The average size of the liposomes according to this invention can vary over a broad range, generally from about 1 nm to about 100 µm. Liposomes or particulate carriers having diameters in the range of about 1 µm and 70 µm are preferred. The person skilled in the art knows that the efficiency of liposomal penetration into the skin increases with decreasing diameter and that therefore liposomes having diameters of about 1 µm to 10 µm, of about 5 to 7 µm or about 5 µm may also be used. Generally the size of liposomes should be selected such that a good penetration into the skin is guaranteed. A particularly preferred embodiment of the invention therefore comprises liposomes having a diameter of between about 1 and 25 µm.

Liposomes in more fluid preparations may be generally more suited for treatment of bacterial infections, while liposomes in more gel-like formulations are generally better suited for treatment of viral infections. It seems that symptoms that are due to viral infections are preferably treated with preparations according to the invention that allow for longer contact times with the affected body areas. Symptoms due to bacterial infections may be treated preferably with preparations that have rather short contact times with the affected body areas.

Thus it is preferred that inventive preparations for the treatment of the various forms of herpes comprise liposomes in a more gel-like preparation such as a gel of medium to high viscosity, waxes or an ointment. Additionally these preparations preferably comprise liposomes of rather large size such as liposomes having a diameter of between about 1 µm and 30 µm, preferably between about 10 µm and 30 µm, more preferably between 20 µm and 30 µm and most preferably at around 25 µm.

Generally, liposomes having a rather small average diameter are better suited for production of solutions, dispersions, suspensions. Such rather small diameters typically comprise diameters of around µm to 10 µm, or even smaller in the case of solutions. In contrast, gel or ointment formulations may comprise liposome of a size of about 1 µm to 50 µm.

Where alternative particulate carriers are used, they are generally prepared as known in the art. Thus, microspheres which are used to deliver a very wide range of therapeutic or cosmetic agents, are made as described for example in WO 95/15118.

Nanoparticles may in some cases be used, provided that they can be loaded with a sufficient amount of active agent and can be administered to the lower respiratory tract according to this invention. They can be prepared according to the methods known in the art, as e. g. described by Heyder (GSF München) in"Drugs delivered to the lung, Abstracts IV, Hilton Head Island Conference, May 1998.

Methods using a pulse laser deposition (PLD) apparatus and a polymeric target to apply coatings to drug powders in a short non-aqueous process are also suitable for the formation of particulate preparations according to this invention. These have e. g. been described by Talton et al., "Novel Coating Method for Improved Dry Delivery", Univ. of Florida UF 1887 (1998).

A further suitable delivery system employs Large Porous Particles as disclosed by David A. Edwards et al. in "Large Porous Particles for Pulmonary Drug Delivery" (Science, 20. June 1997, Vol. 276, p 1868-1871).

Generally, the concentrations in the preparation, particle sizes, active agent loadings etc. will be selected for such alternative carriers to correspond basically to the parameters discussed herein with respect to liposome preparations. Selecting and providing such parameters based inter alia on straightforward experimentation, is well within the skill of an ordinary worker experienced in this art.

According to the invention, use of inventive liposomal preparations is with the treatment of the causes and symptoms, as skin lesions and itchiness, of various herpes forms particularly if the inventive preparations comprise PVP-iodine and liposomes The use of inventive liposomal preparations for treatment of various forms of (herpes?) of varying severity has the advantage that, as only one compound is used, which acts at the same time antivirally and wound-healing promoting, less side effects compared to the use of prior art formulations occur.

The use of the inventive preparations for topical treatment of different forms of herpes allows for efficient and fast treatment of the various forms with significantly reduced side effects and without the formation of scar tissue. A systemic application of the compound, as it is applied during treatment of severe forms of herpes with e.g. nucleoside-analoga, is not necessary. Side effects are thus avoided.

Another advantage of the inventive liposomal PVP-iodine preparations is that they are active against viruses and bacteria. Thus, bacterial inflammatory reactions that may also contribute to the herpes phenotype can be effectively treated during the course of a herpes treatment with the inventive liposomal PVP-iodine preparations. Moreover, a liposomal preparation with an antiseptic compound such as PVP-iodine allows for protracted compound-release from the liposomes. This leads to a prolonged efficacy of the microbial and anti-viral substance and thereby allows for less frequent application of the preparation compared to common antiseptic solutions or nucleoside-analoga preparations.

Preparations according to this invention can take a variety of forms, including pharmaceutically acceptable solid or liquid formulations such as emulsions, dispersions, suspensions, solutions, gels, ointments, waxes, spray, lotions, etc. The formulation as a Hydrogel is preferred. If in the context use is made of the term "gel", this thus always includes the preferred embodiment of a Hydrogel.

Generally, the amount of active agents in an inventive preparation will be determined by the desired effect on the one hand and the carrying capacity of the carrier preparation for the agent on the other hand. Broadly speaking, the amount of active agent in an inventive carrier preparation can range in concentrations between the lower limit of effectiveness of the agent and the maximum loading of the agent in the respective carrier preparation. It is understood that the active compounds are present in the inventive preparations in a pharmaceutically sufficient amount, i.e. in an amount sufficient for treatment of the different forms of herpes.

More specifically, for an antiseptic agent, such as povidone iodine, a solution, dispersion, oil , ointment or gel in an inventive carrier preparation, especially where the carrier is a liposome preparation, can contain between 0.1 and 10 g of agent in 100 g of preparation. Such a preparation will then typically contain between 1 and 5 g of liposome membrane-forming substance, especially lecithin, per 100 g of preparation.

Usually preferred active compound concentrations such as of e.g. PVP-iodine concentration are normally between 1% to 10% and preferably 1% to 5% by weight.

In a lotion, which can be a hydrophilic or a lipophilic lotion, a typical range of active compound such as PVP-iodine will be between 0.5 and 10 g compound, and between 1 and 5 g, preferably about 4 g of liposome membrane forming agent such as hydrogenated soy bean lecithin per 100 g of lotion. In the case of a hydrophilic lotion, electrolyte solution will often be used in preparing the liposome-containing lotion.

A lipophilic lotion will often be made from compound, membrane-forming substance and lipophilic formation agents such as medium chain length triglycerides, etc.

A hydrophilic cream comprising an inventive liposome preparation will generally comprise between 0.1 and 10 g agent, such as PVP-iodine, together with between about 1 and 10 g membrane forming substance and further typical O/W cream forming additives per 100 g of cream.

A comparable amphiphilic cream according to the invention will have similar contents of agent and membrane forming substance such as lecithin, and will have the typical further additives of an amphiphilic cream.

A hydrophilic ointment according to the invention can broadly comprise between 0.1 and 10 g compound and between 1 and 10 g liposome membrane forming substance such as lecithin, together with typical prior art ointment basis substances such as Macrogol (TM) and water in 100 g of ointment.

A non-alcoholic hydrogel according to the invention could broadly comprise between 1 and 5 g compound such as PVP-iodine, approximately 2-4 g lecithin and gel-forming substances such as Carbopol®, with pH-adjusting agent and water to form 100 g of hydrogel.

An inventive aerosol or spray preparation will often comprise up to 50 mg, but could comprise up to and above 100 mg of liposomal active compound formulation per unit spray dose. The spray preparation will typically comprise at least 10 % wt of compound agent such as PVP-iodine in the loaded liposomes (or alternative carrier particles), but may comprise up to 50 % wt or even more of active agent. Where the active agent is PVP-iodine, the amount of available iodine will generally be about 10 % wt (based on PVP-iodine).

More specific formulations are notable from the embodiment examples.

The features and advantages of this invention will become clear in more detail from the ensuing description of preferred embodiments. In these embodiments, which include a best mode, povidone iodine is exemplified as an antiseptic agent and liposomes are chosen as the carrier. This should however not be construed as a restriction of this invention to antiseptic agents alone or, among antiseptic agents, to povidone iodine, and/or to liposomes as the carrier, although such preparations are specifically preferred. According to the invention other particulate carriers such as "large porous particles" or other micelles, nanoparticles, etc. can be formulated with PVP-iodine in order to produce preparations that allow for an efficient treatment of the various forms of herpes. Correspondingly, other halogen-releasing antiseptics can be formulated with liposomes into a preparation that also allows for the efficient topical treatment of herpes. One preferred method for producing the invention's liposomes can generally be described as follows:
The lipid membrane-forming components, e. g. lecithin, are dissolved in a suitable solvent such as chloroform or a 2: 1 mixture of methanol and chloroform and are filtered under sterile conditions. A lipid film is then produced on a sterile high surface substrate, such as glass beads, by controlled evaporation of the solvent. In some cases, it can be quite sufficient to form the film on the inner surface of the vessel used in evaporating the solvent without using a specific substrate to increase the surface.

An aqueous system is prepared from electrolyte components and the (one or more) active agents to be incorporated in the liposome preparation. Such an aqueous system can e. g. comprise 10 mmol/l sodium hydrogen phosphate and 0.9 % sodium chloride, at ph 7.4; the aqueous system will further comprise at least the desired amount of the active agent, which in the embodiment examples is povidone iodine. Often, the aqueous system will comprise an excess amount of agent or agents. The pH of the buffer solutions may be varied.

| | pH 5 | pH 5 | pH 5 | pH 6 | pH 7.4 | pH 7.4 |
|---|---|---|---|---|---|---|
| Na₂HPO₄ | 0.0114 g | | 0.1436 g | | | |
| Na₂HPO₄x2H₂O | | 1.8334 g | | 2.2464 g | 0.445 g | 0.89 g |
| KH₂PO₄ | 0.8970 g | | 0.7973 g | | | |
| Citric acid | | 0.9312 g | | 0.7085 g | | |
| 1 M HCl | | | | | pH adjust. | pH adjust. |
| Water, purified | ad 100 ml | ad 100 ml | ad 100 ml | ad 100 ml | ad 100 ml | ad 100 ml |

Preparations may then finally be adjusted with NaCl or Glycerol to an isotonic range (250-350 mOsmol/kg). Depending on the PVP-iodine concentration the salt strength may also be varied (as e.g. shown for buffer solutions with pH 7.4 in the above table).

The liposomes are generally formed by agitating said aqueous system in the presence of said film formed by the lipid components. At this stage, further additives can be added to improve liposome formation; e. g. sodium cholate. Liposome formation can also be influenced by mechanical action such as pressure filtration through e. g. polycarbonate membranes, or centrifuging. Generally, the raw liposome dispersion will be washed, e. g. with electrolyte solution as used in preparing the above-described solution of the active agent.

When liposomes with the required size distribution have been obtained and washed, they can be redispersed in an electrolyte solution as already described, often also comprising sugars such as saccharose or a suitable sugar substitute. The dispersion can be freeze-dried, and it can be lyophilysed. It can, prior to use, be reconstituted by addition of water and suitable mechanical agitation at the transition temperature of the lipid component, which for hydrogenated soy bean lecithin is e. g. 55 C.

In the following Examples, hydrogenated soy bean lecithin (EPIKURON (TM) 200. SH obtainable from Lukas Meyer, Germany or PHOSPOLIPON (TM) 90 H obtainable from Nattermann Phospholipid GmbH, Germany) was used. However, other pharmaceutically acceptable liposome membrane forming substances can be used instead, and the person skilled in the art will find it easy to select suitable alternative liposome forming systems from what is described in prior art.

The person skilled in the art is well aware that the liposomes-containing dispersion may comprise additional additives and adjuvants that can influence the appearance of the liposomal preparations. The inventive liposomal dispersion may contain e.g. colour pigments that ensure that the slightly yellow or brown colours that are due to PVP-iodine or released iodine are not visible. In the same manner, liposomes or the pharmaceutical liposome-containing preparations may comprise additives that influence the consistency and the smell of the preparations.

The person skilled in the art is well aware that the choice of these additives and adjuvants depends on the intended application form of the preparation (e.g. as ointment, gel or solution) and may be influenced by aesthetic considerations (such as colour and smell).

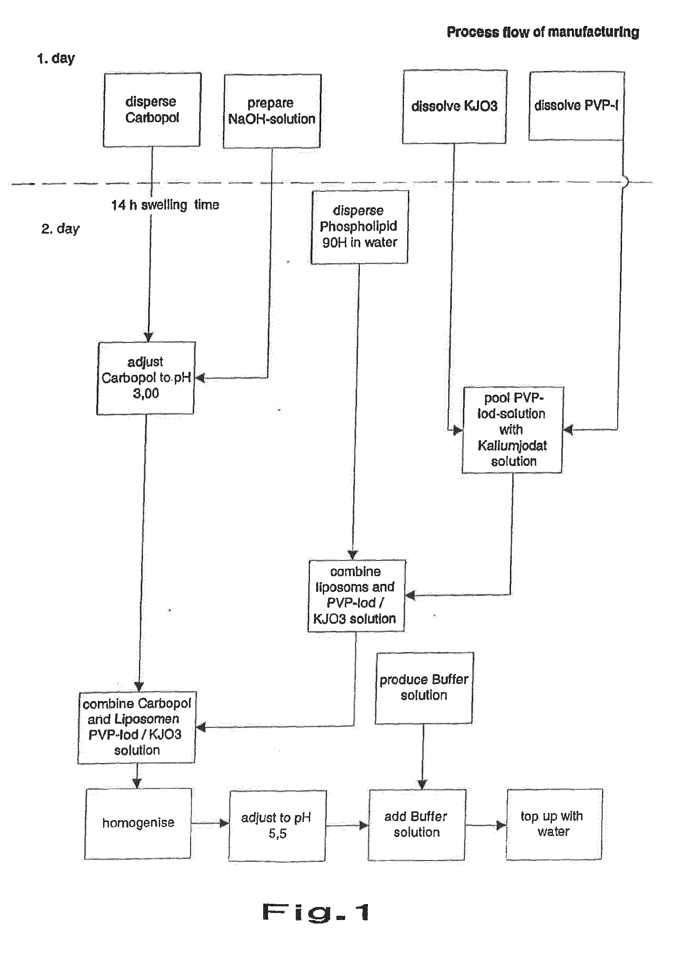

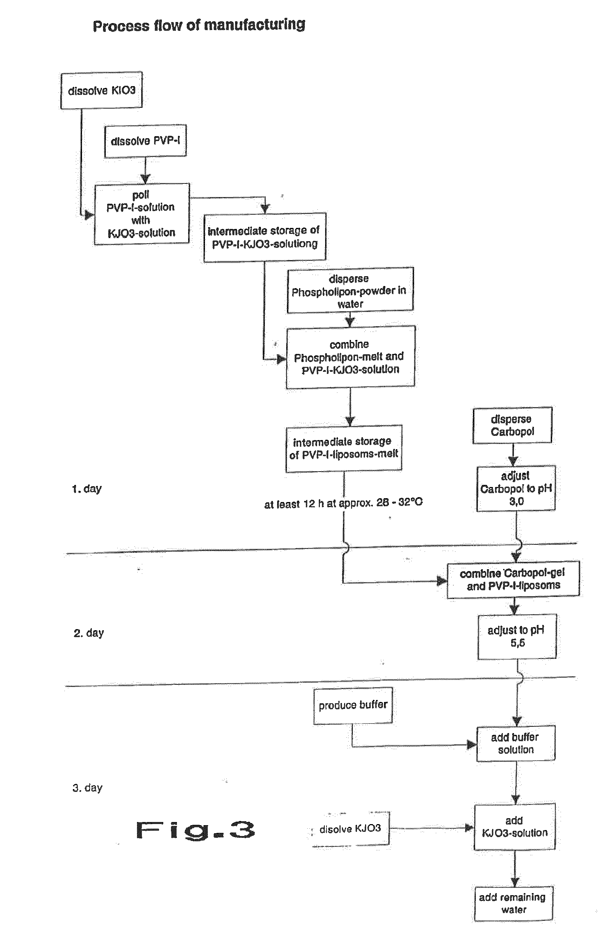

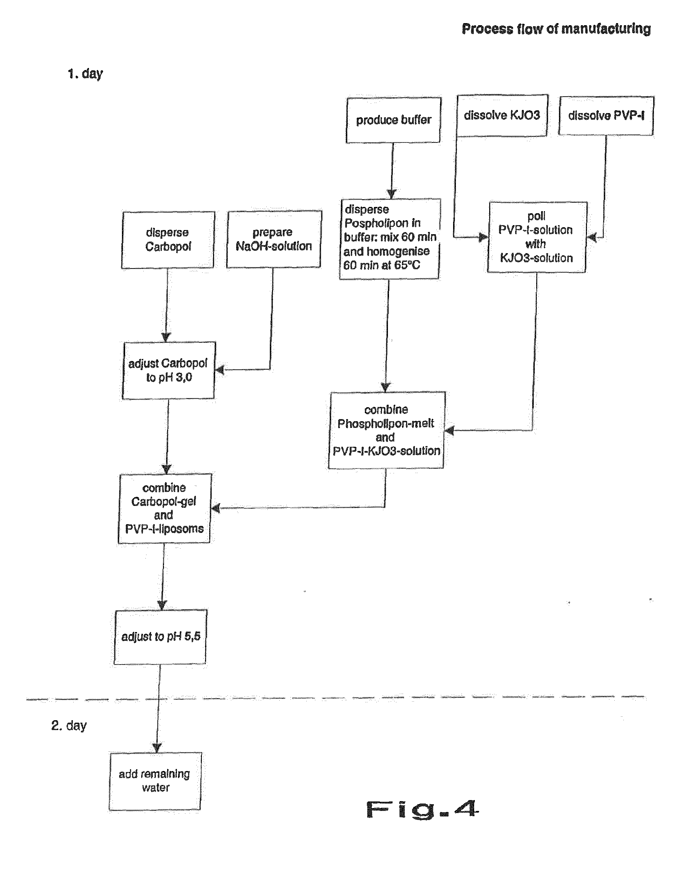

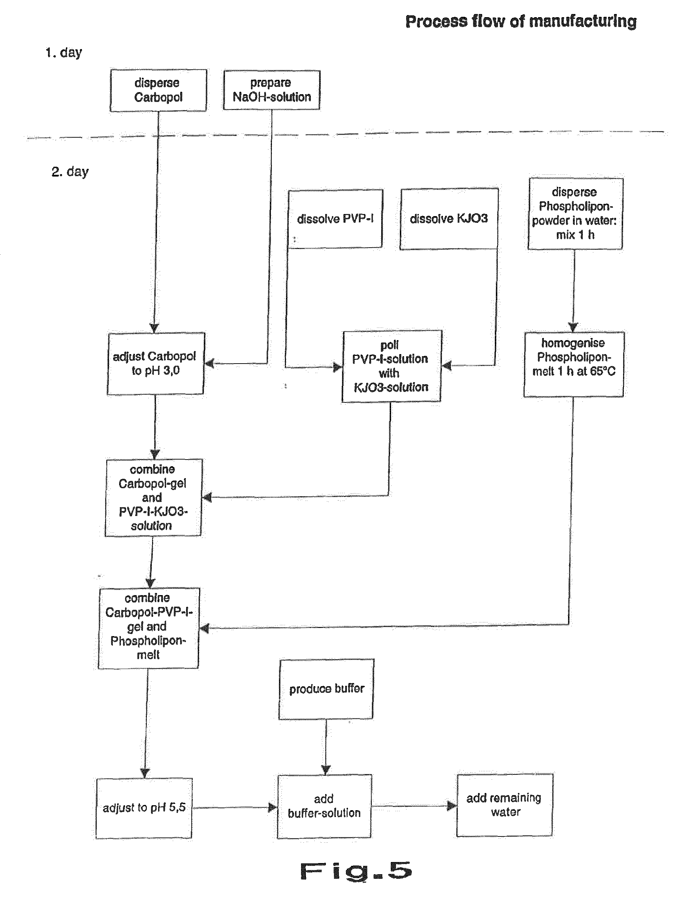

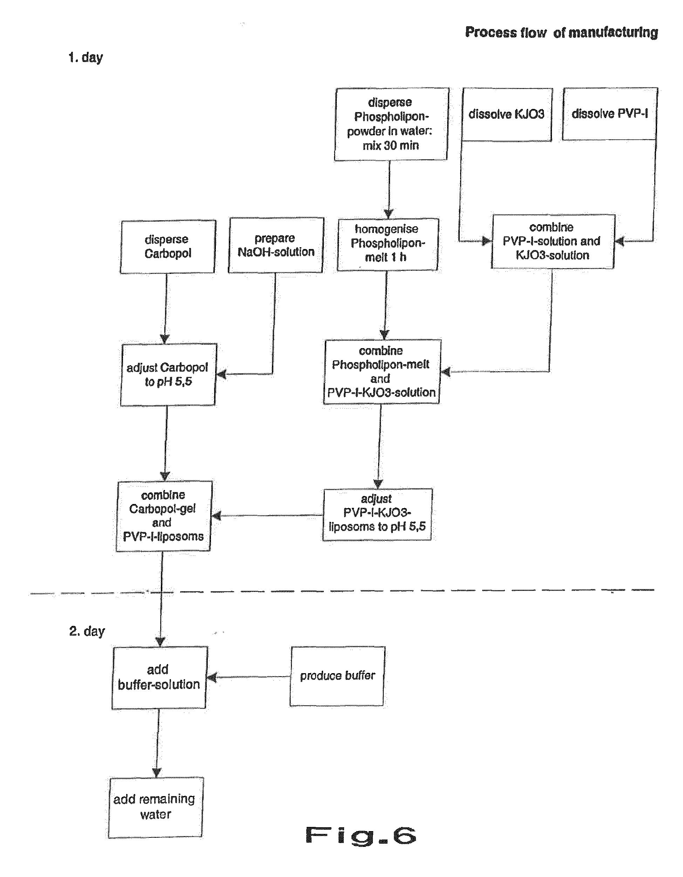

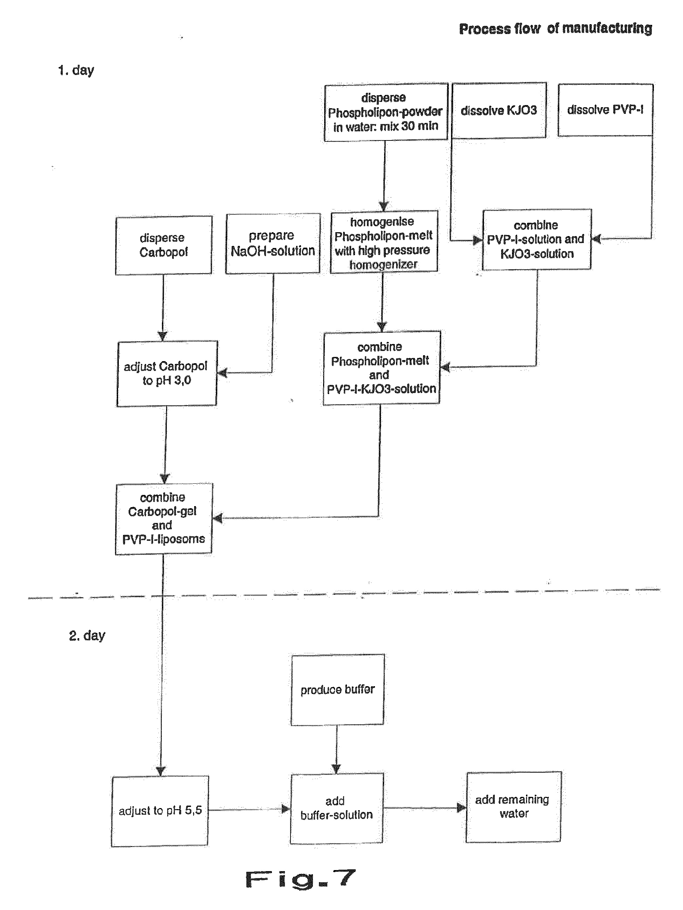

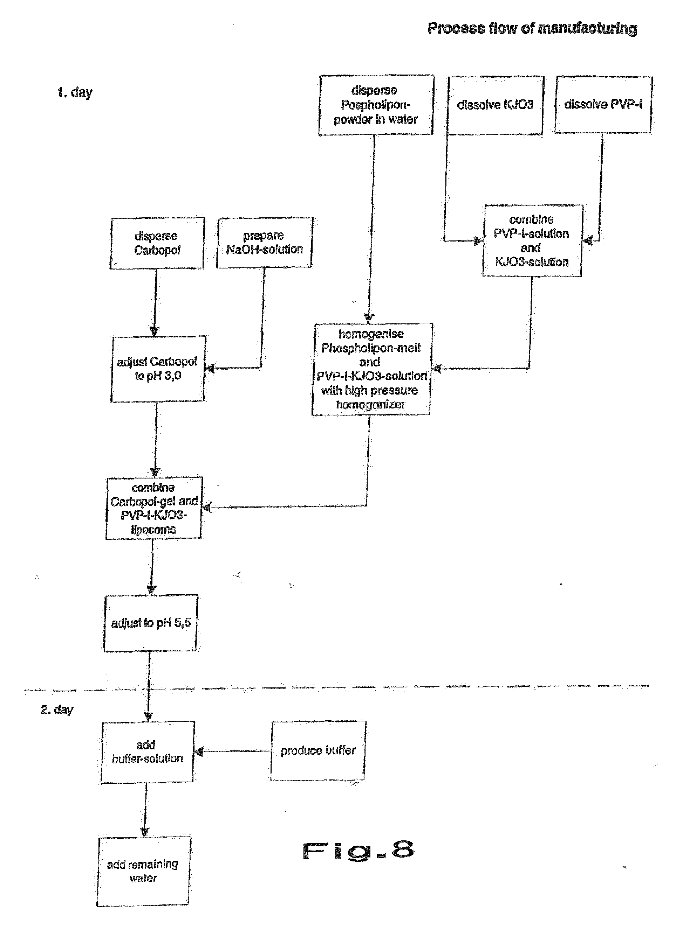

As already mentioned above, a particularly preferred embodiment of the invention is a hydrogel formulation comprising liposomes and PVP-iodine. Such hydrogel formulations usually comprise Phospholipon® 90 H (Aventis, Germany) as liposome-forming material and Carbopol® 980 NF (Noveon Inc., USA) as gel-forming substance. Phospholipon® 90 H is a 90% hydrogenated phosphatidylcholin preparation which is more storage-stable than unhydrogenated phosphatidylcholin preparations. Carbopol® is the trade name for Acrylic acid polymers that are commonly used for formation of pharmaceutically acceptable gels. The preferred hydrogel formulations may also comprise KIO₃, which serves for refurnishing iodine in the preparation. Citric acid and Na₂(HPO₄), which are used as buffering agents, advantageously influence the stability of the preparations. Flow charts of the production process are shown in Figures 1 to 8, with Figure 1 illustrating the currently preferred production process. A detailed discussion of this process is given in Example 6.

Examples are set out below that specifically illustrate the production of preferred embodiments of the invention. They are intended to illustrate how preparations according to the invention may be produced and they should by no means be read as limiting the invention to those examples.

### Embodiment Example I - preparation for in vitro tests

In a 1000 ml glass flask, provided with glass beads for increased surface, 51.9 mg cholesterol and 213 mg hydrogenated soy bean lecithin were dissolved in a sufficient amount of a mixture of methanol and chloroform in a 2:1 ratio. The solvent was then evaporated under vacuum until a film was formed on the inner surface of the flask and on the glass beads.

2.4 g PVP iodine (containing about 10 % available iodine) were separately dissolved in 12 ml water.

Again in a separate vessel, 8.77 g sodium chloride and 1.78 g Na₂HPO₄·2H₂O were dissolved in 400 ml water. Further water was added up to a total volume of 980 ml, and then, approximately 12 ml 1N hydrochloric acid were added to adjust pH to 7.4. This solution was then topped up with water to exactly 1000 ml.

In a fourth vessel, 900 mg saccharose and 57 mg disodium succinate were dissolved in 12 ml water.

The PVP iodine solution was then added to the lipid film in the flask and the mixture was shaken until the film dissolved. The resulting liposome formulation was separated from the hydrated lipids in the flask. The product was centrifuged and the supernatant liquid was discarded. The saccharose solution was added ad 12 ml and the product was again centrifuged. Afterwards the supernatant liquid was again discarded. At this stage, a further washing step, using the saccharose solution or the sodium chloride buffer solution could be carried out.

After the last centrifugation step and discarding of the supernatant, 12 ml sodium chloride buffer solution was added, and the liposomes were homogenously distributed therein. The product was then distributed into vials each containing 2 ml liposome dispersion, and the vials were then subjected to a freeze-drying step.

After the freeze-drying, each vial comprised about 40 mg solids.

The method of Embodiment Example I has a minor disadvantage in that the PVP iodine solution used, due to the high percentage of solids, is rather viscous and thus more difficult to handle.

### Embodiment Example II

In a 2000 ml flask provided with glass beads to increase surface, 173 mg hydrogenated soy bean lecithin and 90 mg disodium succinate were dissolved in approximately 60 ml of a methanol/chloroform mix in a 2:1 ratio. The solvent was removed under vacuum until a film was formed.

4 g PVP iodine (10 % available iodine) were dissolved in 40 ml of the sodium chloride buffer solution described in Embodiment Example I, and were added to the lipid film in the flask. The flask was then shaken until the film dissolved and liposomes were formed.

The product was centrifuged and the supernatant liquid was discarded.

To the thus produced liposome pellet, further 40 ml sodium chloride buffer solution was added, and the centrifuging step was repeated. The supernatant was again discarded. At this stage, the washing step could be repeated where necessary.

After the final centrifuging and decanting step, 40 ml sodium chloride buffer solution was again added to the precipitated liposomes. The homogenous dispersion was then distributed into vials, each vial containing about 2 ml liposome dispersion, and the vials were then subjected to a freeze-drying step. This produced approximately 200 mg freeze-dried solids per vial.

From the freeze-dried solids of Examples I and II, further preparations were made as described in subsequent Embodiment Examples and Test Reports.

Like that of Embodiment Example I, the above-described method uses a hydrating step after film formation in the presence of organic solvents and aims at inclusion rates of 5 to 15 %. These methods generally produce rather large and often multilamellar liposomes.

The above-described methods can be modified by a high pressure filtering step through a suitable membrane such as a polycarbonate membrane after the raw liposomes have been formed or after any of the subsequent washing steps or directly by using high pressure homogenization. This produces much smaller, unilamellar liposomes at increased amounts of encapsulated agent.

Instead of high pressure homogenization, other prior art methods known to provide small uniform sized liposomes can be employed.

### Embodiment Example III

A hydrophilic (O/W) cream was prepared from 10 g hydrogenated soy bean lecithine/PVP iodine liposomes as described in Embodiment Example II; these were mixed with 4 g Polysorbate 40 (TM), 8 g cetylstearyl alcohol, 8 g glycerol, 24 g white vaseline, and water ad 100 g.

### Embodiment Example IV

An amphiphilic cream was prepared from 10 g hydrogenated soy bean lecithine/povidone iodine liposomes as described in Embodiment Example II; 7.5 g medium chain length tryglyceride, 7 g polyoxyethyleneglycerol monostearate, 6 g cetylstearyl alcohol, 8 g propylene glycol, 25 g white vaseline, and water ad 100 g.

### Embodiment Example V

A hydrophilic ointment which can be rinsed off with water was prepared using 10 g of liposomal PVP iodine as described in Embodiment Example II, 55 g Macrogol 400 (TM), 25 g Macrogol 4000 (TM), and water ad 100 g.

### Embodiment Example VI

A hydrogel was prepared from 4 g liposomal PVP iodine as described in Embodiment Example II, 0.5 g Carbopol® 980 NF (TM), sodium hydroxide ad pH 7.0, water ad 100 g. Further modifications of the above-described embodiments are envisaged.

Thus, the creams of Embodiment Examples IV and V can have an additional content of an agent known to promote the healing of wounds, such as allantoin.

Such an agent will be added in a pharmaceutically useful concentration, in the case of allantoin in the range of 0.1 to 0.5 g, per 100 g of cream. The wound healing agent can be incorporated in the cream base, in which case it will largely be outside the liposomes. It can, however, be partly or mostly incorporated in the liposomes, in which case it will be added at a corresponding suitable stage of the liposome preparation method.

Similar alternatives are easily envisaged on the basis of the further Embodiment Examples.

It is also possible to prepare embodiments similar to the above described ones, which comprise an agent capable of promoting the healing of wounds instead of, and not in addition to, the antiseptic agent as e. g. povidone iodine disclosed in the above Embodiment Examples. Presently, it is however preferred to use a wound healing promoting agent (if at all) in addition to an antiseptic agent.

For application of the inventive preparations to a patient, known systems can be used, such as pneumatic pump applicators, two-chamber gas pressure packs, aerosol spray dispensers etc.

In a pneumatic pump applicator, a bellows device is provided between an upstream and a downstream valve, both valves operating one way in the same direction. A supply of pharmaceutical preparation, such as an ointment or gel, is contained in a reservoir upstream of the valves-and-bellows device.

When compressing the bellows, the downstream valve opens and permits a dosed amount of preparation to leave the device for application. When the bellows is extended, this valve shuts and prevents reentry of the preparation. At the same time, the upstream valve opens and permits preparation from the reservoir to enter into the bellows, for release through the downstream valve upon the next compression step of the bellows.

The reservoir is sealed by a closure element which can move through the reservoir like a piston moves in a cylinder. By the stepwise emptying of the reservoir, this closure element is sucked into the reservoir, so that the remaining amount of pharmaceutical preparation in the reservoir is always sealed off, while at the same time the reservoir can be emptied. Such a device is useful for pasty preparations, creams, ointments etc.

In a two-chamber gas pressure pack, the pharmaceutical preparation is contained in a bag of flexible plastics film material. Often, this is high pressure polyethylene.

The bag is contained inside a gas tight pressure vessel which further contains a supply of pressurizing gas, very often a compressed inert gas like nitrogen or air. The plastic film bag has only one outlet, which is gas-tightly connected to the interior wall of the pressure vessel, surrounding a single opening thereof. The pressurized gas in the vessel tends to compress the bag, driving the pharmaceutical preparation inside the bag out through the opening of the bag and thus through the opening of the vessel. A valve and, in case, spray-head device is provided in the vessel mouth. Operating the valve releases a spray mist, a jet of liquid or a portion of flowable solid such as cream. Using such a system, solutions, emulsions, creams, oitments and gels can be dosed and applied.

### Embodiment example VII

A Hydrogel was formulated according to the flow chart shown in figure 1. The amounts shown in Table 1 were used either for analytical or scale up preparations.

**Table I**

| Pos. | Substance | Amount (g/100g) | Scale up (kg/1500kg) |
|---|---|---|---|
| A | H₂O | 15,0 | 200,0 |
| A | Phospolipon 90 H | 3,0 | 45,0 |
| B | H₂O | 40,0 | 600,0 |
| B | Carbopol® 980 NF | 1,5 | 22,5 |
| C | H₂O | 2,0 | 30,0 |
| C | KIO₃ | 0,0708 | 1,09 |
| D | H₂O | 20,0 | 300,0 |
| D | PVP-iodine 30/06 Avalaible iodine (10%) | 3,0 | 45,0 |
| E | H₂O | 2,5 | 50,0 |
| F | H₂O | 2,5 | 50,0 |
| G | H₂O | 4,6 | 69,0 |
| G | NaOH solid | 0,46 | 6,9 |
| I | Citric acid, H₂O free | 0,1065 | 1,059 |
| I | Na₂(HPO)₄, H₂O free | 0,225 | 3,37 |
| I | H₂O | 3,0 | 45,0 |
| H | H₂O | ad 100,0 | ad 1500 |

Pos. stands for Position (see also below Table 2). Phospholipon® 90 H was purchased from Aventis (Germany). Carbopol® 980 NF was purchased from (Noveon Inc., USA) and PVP Iodine 30/06 was purchased from BASF (Germany).

In Table 2, column 2 the exact order of steps and the parameters of each step are given (see also Figure 1). Column 3 discusses non-exclusive alternatives. All steps were performed at room temperature except where indicated otherwise. All substances were of a purity grade common for pharmaceutical preparations such as described in the British Pharmacopeia.

**Table II**

| No. | embodiment example VII | Alternatives |
|---|---|---|
| 1 | Carbopol 980 NF is mixed into H₂O without agglomeration (Pos. B). Stirring for 30 min at approx. 30 upm (units per minute) in conventional stirrer. Visual control for Polyacrylic acid-agglomerates. | Substances: Other gel-forming substances may be used. |
| | | |
| | | Homogenization time can vary: |
| | | shorten to 1min |
| | | prolong to 10 min |
| | If necessary, homogenize gel in conventional homogenisator for 2 min at 3000 upm. | (caution! gel structure may be destroyed) |
| | | Stirring time can be altered as desired. Only condition is that gel is free of agglomerates at the end. |
| | Subsequently stir gel for 30 min at 30 upm in conventional stirrer. Eventually control again for Polayacrylicacid-agglomerates | |
| | | |
| | | Swelling time may be altered from 15 min to 5 days. Preferably the gel has formed before other substances are added. |
| | If present, remove them and stir again for 15 min at 30 upm. Eventually homogenize again. | |
| | | |
| | | Adjustment of pH to 2-8 may be performed at this stage. Adjustment to pH 3-6 is preferred. |
| | Let gel swell for at least 14 h. | |
| 2 | Dissolve H₂O and KIO₃ completely in a suitable vessel (Pos. C). A 30-40% KIO₃-solution may also be used. | H₂O-temperature may be adjusted to anywhere between ambient temperature and 100 °C. |
| | | |
| | | KIO₃ is not obligatory. |
| 3 | Dissolve NaOH completely in H₂O (Pos. G). | NaOH is used in concentrations common for pharmaceutical preparations. |
| | | |
| | | Other Bases or substances suggested by the supplier of the gel forming substances may also be used for formation of gel structure as e.g. KOH, Triethanol-amine, 2-Amino-2-methyl-1-propanol, tris(hydroxymethyl)aminoethan, 2-hydroxypropy)-ethylen-diamine, diisopropanolamine |
| 4 | Mix PVr-iodine into H₂O while stirring at 1000 upm in conventional stirrer (Pos. D). | Stirr ng time and speed can be altered arbitrarily. |
| | | |
| | | Important: PVP-Iodine has to be dissolved completely. |
| | Stir mixture for futher 60-70 min at 1000 upm until it is comoletely dissolved. | |
| 5 | Warm H₂O to 65 °C while stirring with 1000 upm in conventional stirrer. Then add slowly Phospholipon® 90 H (Pos. A). Take care that no agglomerates are formed. | Possible temperature range: 40 °C - 120 °C. 50 °C - 75 °C is preferred because of phase transition temperature. |
| | | |
| | | Other liposome-forming materials or mixtures thereof may be used. |
| | Stir dispersion for further 90 min at 65 °C-70 °C and 1000 upm. | |
| | | |
| | | Stirring time and speed: Is dependent on equipment. A complete dispersion has to be achieved. Apparatus of the rotor/stator principle, high pressure homogenisators, ultrasound or extrusion technology may also be used for stirring. |
| | Subsequently cool liposomal dispersion to ≤ 30 °C while stirring at 500 upm | |
| 6 | By adding the NaOH-solution (No. 3) the gel is adjusted to a pH of 3.0 (± 0,2). | Further processing to a gel may be feasible without pH pre-adjustment and is dependent on the gel-forming substance |
| 7 | The KIO₃ solution (No.2) is added to the PVP-Iodine solution (No. 4) while stirring at 1000 upm. | Reaction between KIO₃ and PVP-iodine is time dependent. To ensure a complete reaction, the stirring time has to adapted accordingly. Thus, stirring time may be between 10 min and 2 h |
| | | |
| | Stirring continued for at least 60 min. | |
| 8 | The PVP-iodine- KIO₃ -solution is pumped into the liposomal dispersion (No. 5). | stirring time is variable depending on until when an homogeneous mixture has formed. |
| | | |
| | Subsequently it is stirred for 30 min at 1000 upm. | |
| 9 | The PVP-iodine- KIO₃-liposomes-dispesion is added to the gel (No. 6). | Stirring time is variable depending on until when an homogeneous mixture has formed. |
| | | |
| | It is stirred for 30 min a 30 upm. | Stirring time should be as short as possible so that gel structure gets not disrupted. |
| | | |
| | ***Subsequently homogenization is performed by forced circulation pumping for 2 min at 2800 upm*.** | |
| | | |
| | After checking for agglomerates, it may be homogenized for further 1 - 2 min. | |
| 10 | Remove agglomerates if present. | Adjust stirring time and speed to gel quality. |
| | | |
| | Add 50,0 kg NaOH-solution (in the scale up, point 3) while stirring at 30 upm. | Amounts of NaOH may vary. Adding of base by step wise adjustment until desired pH is achieved. |
| | | |
| | Stir for further 30 min at 30 upm at ≤30 °C. | |
| | Cool if necessary. | |
| | | |
| | Determine pH and add additional NaOH until an pH of 5.5 (±0.2) is achieved. After each adding step stir for 20 min. | |
| | | |
| | After each adding step homogenize by circulation pressure pumping for 15 sec at 1000 upm. | |
| | | |
| | After adjustment of pH stir for further 15 min at 30 upm. Check pH and correct if necessary. | |
| | | |
| | After successful pH adjustment add remaining H₂O amount which depends on the amount of NaOH used. | |
| 11 | Mix buffer solution at 30 °C while stirring until it is completely dissolved (Pos. I). | Temperature can be raised to 40 °C. Other suitable buffers may also be used. |
| 12 | Buffer solution is added to the product (No. 10) while stirring for 15 min at 30 upm. | The desired product quality (storage stability) is achieved by addition of the buffer. |
| | | |
| | Degas by application of vacuum. | Stirring time is variable depending on until when an homogeneous mixture has formed. |
| | | |
| | | Degasing may be achieved by other means than vacuum. |
| 13 | Add the remaining H₂O-amount (Pos. H) and stir for 30 min at 25 upm | stirring time is variable depending on until when an homogeneous mixture has formed. |
| | Optionally homogenization may be performed by circulation pressure pumping for 15 sec at 1000 upm. | |
| | | |
| | Stir for another 30 min. | |
| | | |
| | Check visually for agglomerates | |

Positions E and F of Table I are used for washing the KIO₃- and PVP-iodine vessels (points 2 and 4 of Table II).

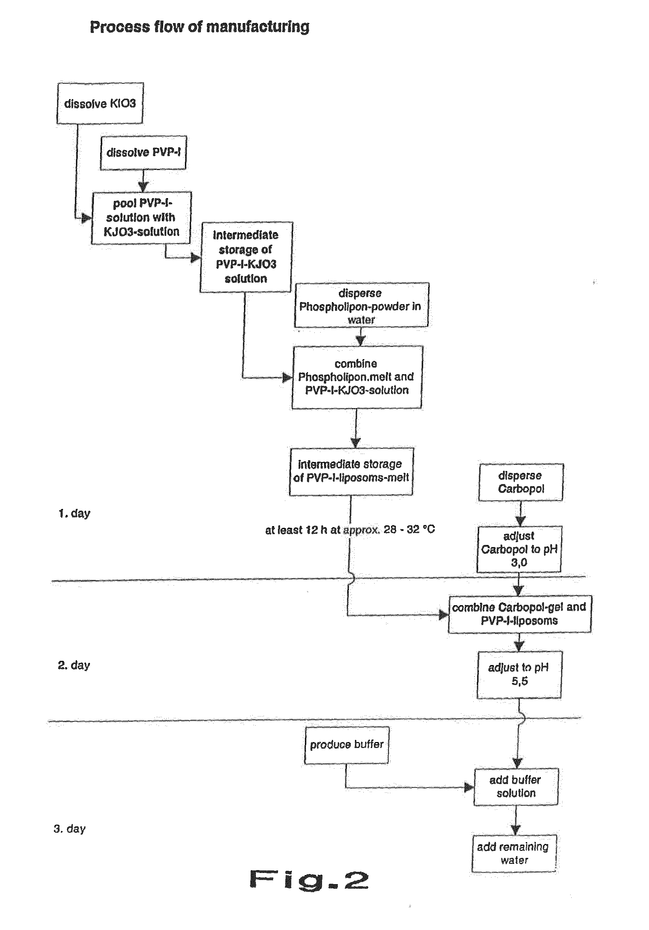

As mentioned above, the Hydrogel-Formulation is produced according to the method set out in Table 2 and Figure 1. Alternative methods become obvious from the flow charts of figures 2 to 8. The individual steps may be performed as set out above.

Using inventive preparations efficiency tests were then carried out, as follows:

### Test I

This was an in-vitro-test of the bactericidal effect provided by an inventive povidone iodine liposome preparation. The test was based on the quantitative suspension test as described in "Richtlinien der Deutschen Gesellschaft für Hygiene und Mikrobiologie", 1989. In this test, the bactericidal agent is used to kill staphylococcus aureus (ATCC 29213), a major problem in hospital hygiene.

The liposome preparation used was that of Embodiment Example I. At different contact times between 1 und 120 minutes, the minimum concentration of the preparation in water was determined which was capable of killing the staphilococci.

The results are shown in Table 3.

**TABLE III**

| Contact Time (Minutes) | Bactericidal Concentration |
|---|---|
| 1, 2, 3, 4 | ≥ 0.060% |
| 5, 30, 60 | ≥ 0.015% |
| 120 | ≥ 0.007 % |

The results show that at short contact times (between 1 and 4 minutes) the bactericidal concentration is as low as 0.06 % and that at long contact times (120 minutes) the bactericidal concentration can be as low as 0.007 %.

### Test II

The virucidal and chlamydicidal activity of liposomal PVP-iodine has been studied, in cell cultures, by Wutzler et al., 9th European Congress for Clinical Microbiology and Infection Diseases, Berlin, March 1999. In cell cultures, liposomal PVP-iodine is highly effective against herpes simplex virus type 1 and adenovirus tpye 8, while the long-term cytotoxicity experiments indicated that the liposomal form is better tolerated than aqueous PVP-iodine by the majority of cell lines tested. PVP-iodine in liposomal form is not genotoxic.

### Test III

A 3% PVP-iodine hydrogel liposomal preparation was compared with a 3% PVP-iodine ointment, where the active agent was not in liposomal form. The agent was applied to standardized in vitro cultures of rat skin and peritoneal explants, as a screening for tissue compatibility of skin and wound antiinfectives.

The growth rate of the cultured explants was studied after 30 minutes exposure and incubation with a test substance.

Again, the substantially better toleration of the liposomal preparation was clearly shown in the results, in terms of peritoneum growth rate and skin growth rate.

With the ointment, the peritoneum growth rate reached 85%, and the skin growth rate reached 90%; with the liposomal hydrogel formulation, the peritoneum growth rate was 96%, and the skin growth rate was 108%; these values are to be compared with 100% values in a control test using Ringer's solution as the agent.

### Test IV

The toleration of liposomal PVP-iodine solutions for nasal applications was studied by investigating the influence of different test substances on ciliated epithelium cells, the most sensible cells of the mucous membrane. A cytotoxic damage of these cells which would cause a restriction of the mucociliar clearance can be determined by a detectable decrease of the ciliary vibration.

Human ciliated epithelium cells were analysed by an in-vitro method which enables the determination of the ciliary activity or ciliary vibration. The corresponding cells were exposed and incubated with 100 je. 1 test substance at a temperature of 37 C. After an incubation period of 5 minutes the ciliary vibration was measured.

By using this in-vitro method a nutriant solution (Dulbeco) as standard, a 0.2% chlorohexidine solution (typical antiseptic agent), conventional polyvidone iodine solutions (Betaisodona) of different concentrations (5.0%, 2.5% and 1.25% PVP-iodine) and a liposomal solution containing 4.5% of PVP-iodine were tested.

The substantially better toleration of the liposomal preparation was clearly shown in the results: if the ciliated epithelium cells were exposed to the Betaisodona solutions containing 5.0% or 2.5% PVP-iodine, no ciliary activity could be observed after the incubation period. Treating the cells with a chlorohexidine solution led to a decrease of the measured ciliary vibration in comparison to the standard (nutriant solution). The low concentrated Betaisodona solution containing 1.25% PVP-iodine, didn't cause a detectable decrease of the ciliary activity. With respect to the measured ciliary vibration no differences to the standard (nutrian solution) could be determined by exposing the human ciliated epithelium cells to the concentrated liposomal 4.5% PVP-iodine solution.

These results indicate that the liposomal formulation is well tolerated for nasal application and advantageous with respect to for e. g. chlorohexidine or conventional Betaisodona solutions.

In view of the above, the present application also relates to the following numbered aspects:
1. Method for producing a pharmaceutical preparation for the treatment of Herpes,
   characterized in, that the preparation comprises at least one antiseptic compound in a pharmaceutically effective amount combined with a particulate, pharmaceutically acceptable carrier.
2. Method according to aspect 1,
   characterized in, that the particular carrier comprises liposomes, microspheres, nanoparticles, "Large Porous Particles", laserpuls-polymer coated molecules particles and/or other micelles.
3. Method according to aspect 1 or 2,
   characterized in, that the antiseptic compound comprises oxygen- and halogen-releasing compounds, preferably iodine and iodine complexes, and/or metal compounds, preferably silver- and mercury-compounds.
4. Method according to aspect 3,
   characterized in, that the antiseptic compound is PVP-iodine.
5. Method according to one of aspects 1 to 4,
   characterized in, that the preparation comprises additional antiseptic compounds such as organic disinfectants including formaldehyde-releasing compounds, phenolic compounds including alkyl- and aryl-phenolic compounds, chinolines and acridines, hexahydropyrimidines, quartenary ammonia compounds, imines and salts thereof and guanidines.
6. Method according to one of aspects 1 to 5,
   characterized in, that the preparation comprises additionally wound-healing promoting agents, that promote granulation and epithel-formation such as dexpanthenol, allantoines, azulenes, tannins, vitamins, preferably vitamin B and derivatives thereof.
7. Method according to one of the preceding aspects,
   characterized in, that the particulate carriers, particularly liposomes, have a size in a range between approximately 1 µm and approximately 100 µm, preferably between approximately 1 µm and approximately 50 µm and most preferably between approximately 1 µm and approximately 25 µm.
8. Method according to one of the preceding aspects,
   characterized in, that the particulate carriers, particularly liposomes, release the active compound(s) over an extended time period, preferably over an time period of several hours duration.
9. Preparation according to aspect 8,
   characterized in, that the particulate carriers, particularly liposomes, release the active compound(s) at approximately the same release rate over the time of the release.
10. Method according to one of the preceding aspects,
   characterized in, that the preparation comprises additives and adjuvants such as conserving agents, antioxidants and consistency-forming additives.
11. Method according to one of the preceding aspects,
   wherein the preparation is provided in the form of a solution, suspension, dispersion, ointment, spray, lotion, cream, gel or hydrogel comprising the compound-loaded particulate carriers, preferably in the form of a liposomal solution, suspension, dispersion, ointment, lotion, cream, gel or hydrogel.
12. Method according to one of the preceding aspects,
   wherein the preparation is provided in the form of a pharmaceutical solution-, suspension-, dispersion-, ointment-, lotion-, cream-, gel- or hydrogel-formulation that comprises:
   - liposomes comprising a pharmaceutically acceptable liposomal membrane forming substance and
   - a 0,1% to 5% PVP-iodine solution (with approximately 10% available iodine in the PVP-complex,
   wherein the liposomes are of a size with diameters between approximately 1 µm and approximately 50 µm and, in case, the formulation additionally comprises customary additives, adjuvants and auxiliary substances of a pharmaceutical solution-, suspension-, dispersion-, ointment-, lotion-, cream-, gel- or hydrogel-formulation.
13. Method according to aspect 12,
   characterized in, that the lipsomes are of a size with diameters between approximately 1 µm and approximately 25 µm.
14. Method according to one of the preceding aspects,
   wherein the preparation is suitable for the treatment of Herpes, particularly for the symptoms of Herpes infections that are due to infections with Herpes viruses such Herpes simplex virus Typ I and/or Typ II or Herpes zoster.
15. Method according to aspect 14,
   wherein the preparation is suitable for the treatment of *Herpes labialis, Herpes genitalis, Herpes febrilis, Herpes solaris, Herpes menstrualis* and/or *Herpes traumatica.*
16. Method according to aspect 14,
   wherein the preparation is suitable for the treatment of shingles, facial erysipelas, chicken pox or other inflammatory skin diseases that are provoked by Herpes zoster viruses.
17. Method according to one of the preceding aspects,
   wherein the preparation can be used for the topic treatment of the various symptoms and causes of the various Herpes forms, preferably for treatment of skin damages and blisters in the face, on the lips, in the breast and genital areas and at the extremities as well as for suppression of itchiness.
18. Method according to one of the preceding aspects,
   wherein the preparation can be used for the topic treatment of the bacterial and viral inflammations and infections that occur in the course of the different Herpes forms.

## Claims

1. Use of iodine or at least one iodine complex, which contains the iodine in elementary form, for the manufacture of a pharmaceutical preparation for the treatment of Herpes,
**characterized in that** the preparation comprises said iodine or said at least one iodine complex in a pharmaceutically effective amount combined with pharmaceutically acceptable liposomes.

2. Use according to claim 1,
**characterized in that** the at least one iodine complex is PVP-iodine.

3. Use according to any of claims 1 to 2,
**characterized in that** the preparation comprises additionally wound-healing promoting agents, that promote granulation and epithel-formation such as dexpanthenol, allantoines, azulenes, tannins, vitamins, preferably vitamin B and derivatives thereof.

4. Use according to one of the preceding claims,
**characterized in that** the liposomes have a size in a range between 1 nm and 100 µm, preferably between 1 µm and 50 µm and most preferably between 1 µm and 25 µm.

5. Use according to one of the preceding claims,
**characterized in that** the liposomes release the active compound(s) over an extended time period, preferably over a time period of several hours duration.

6. Use according to one of the preceding claims,
**characterized in that** the liposomes release the active compound(s) at the same release rate over the time of the release.

7. Use according to one of the preceding claims,
**characterized in that** the preparation comprises additives and adjuvants such as conserving agents, antioxidants and consistency-forming additives.

8. Use according to one of the preceding claims,
wherein the preparation is provided in the form of a solution, suspension, dispersion, ointment, spray, lotion, cream, gel or hydrogel comprising the iodine- or iodine complex-loaded liposomes.

9. Use according to one of the preceding claims,
wherein the preparation is provided in the form of a pharmaceutical solution-, suspension-, dispersion-, ointment-, lotion-, cream-, gel- or hydrogel-formulation that comprises:
• liposomes comprising a pharmaceutically acceptable liposomal membrane forming substance and
• a 0,1 % to 5% by weight PVP-iodine solution,
wherein the liposomes are of a size with diameters between 1 µm and 50 µm and, in case, the formulation additionally comprises customary additives, adjuvants and auxiliary substances of a pharmaceutical solution-, suspension-, dispersion-, ointment-, lotion-, cream-, gel- or hydrogel-formulation.

10. Use according to claim 9,
**characterized in that** the liposomes are of a size with diameters between 1 µm and 25 µm.

11. Use according to one of the preceding claims,
wherein the preparation is particularly suitable for the treatment of symptoms of Herpes infections that are due to infections with Herpes viruses Type I and/or Type II or Herpes zoster.

12. Use according to claim 11,
wherein the preparation is suitable for the treatment of *Herpes labialis, Herpes genitalis, Herpes febrilis, Herpes safaris, Herpes menstratalis* and/or *Herpes traumatica.*

13. Use according to claim 11,
wherein the preparation is suitable for the treatment of shingles, facial erysipelas, chicken pox or other inflammatory skin diseases that are provoked by Herpes zoster viruses.

14. Use according to one of the preceding claims,
wherein the preparation can be used for the topic treatment of the various symptoms and causes of the various Herpes forms, preferably for treatment of skin damages and blisters in the face, on the lips, in the breast and genital areas and at the extremities as well as for suppression of itchiness.

15. Use according to one of the preceding claims,
wherein the preparation can be used for the topic treatment of the bacterial and viral inflammations and infections that occur in the course of the different Herpes forms.
